Europäisches Patentamt

European Patent Office

Office européen des brevets

(19)

(11) Publication number : **0 466 659 A2**

## (12) EUROPEAN PATENT APPLICATION

(21) Application number : **91830223.3**

(51) Int. Cl.⁵ : **A61B 17/14, A61B 17/56**

(22) Date of filing : **28.05.91**

(30) Priority : **22.06.90 IT 2072290**

(43) Date of publication of application :
**15.01.92 Bulletin 92/03**

(84) Designated Contracting States :
**BE CH DE ES FR GB LI NL SE**

(71) Applicant : **G. CREMASCOLI S.R.L.**
**Via Clemente Prudenzio, 14/16**
**I-20138 Milano (IT)**

(72) Inventor : **Ghisellini, Franco, c/o G.**
**CREMASCOLI S.r.l.**
**Via Clemente Prudenzio, 14/16**
**I-20138 Milano (IT)**

(74) Representative : **Cicogna, Franco**
**Ufficio Internazionale Brevetti Dott.Prof.**
**Franco Cicogna Via Visconti di Modrone, 14/A**
**I-20122 Milano (IT)**

(54) **Apparatus for resecting the femoral and tibial bones for fitting knee articulation prostheses.**

(57)    An apparatus for resecting the femoral and tibial bones for fitting knee articulation prostheses comprises a slide supporting central body, therewith a sliding guide for the distal resection of the femoral condyles and a top body for the front femoral resection cooperate.
   The apparatus further comprises a dihedral body for forming guide slots for the real femoral resection and for required tapering subsidiary resections.

EP 0 466 659 A2

Jouve, 18, rue Saint-Denis, 75001 PARIS

FIG.1

## BACKGROUND OF THE INVENTION

The present invention relates to an apparatus for resecting the femoral and tibial bones for fitting knee articulation prostheses to the resected bones.

As is known, in the case of diseases of a knee articulation, due to fractures, arthritis, tumural affections or other causes, it is necessary to frequently use replacement articulation prostheses either of a full or of a partial type.

Also known is the fact that, for applying these prostheses, it is necessary to resect the bottom end of the fermoral bone, in order to properly apply said prostheses.

For performing the above mentioned resection operations, there are already available resecting apparatus which must be properly arranged at the bottom end portion of the femoral bone, so as to provide reference and guide points, in order to properly locate resecting blade elements.

These apparatus, however, are affected by some application drawbacks and, moreover, they are not adapted to provide, under any operating conditions, a required application accuracy.

## SUMMARY OF THE INVENTION

Accordingly, the aim of the present invention is to overcome the above mentioned drawbacks, by providing an apparatus adapted to facilitate the resecting operations of the femoral and tibial bones for fitting knee articulation prostheses to the resected bones, which apparatus is adapted to be easily and quickly mated to the different morphologic shapes of the femoral bone, so as to provide, under any operating conditions, a proper resection of the bone itself

Within the scope of the above mentioned aim, a main object of the present invention is to provide such an apparatus which can be easily and quickly fitted, under any operating conditions,to the slope, with respect to a vertical axis, of the diaphysis axis of the femoral bone of the patient.

Another object of the present invention is to provide such an apparatus for facilitating the femoral resection operations which is very reliable and safe in operation.

Yet another object of the present invention is to provide such an apparatus which can be easily made starting from easily available elements and materials.

According to one aspect of the present invention, the above mentioned aim and objects, as well as yet other objects, which will become more apparent hereinafter, are achieved by an apparatus for resecting the femoral and tibial bones for fitting knee articulation prostheses to the resected bone, characterized in that said apparatus comprises a slide bearing central body, therewith a sliding guide, for distally resecting the femoral condyles, and a top body, for performing the front femoral resection cooperate, there being moreover provided a dihedral body adapted to form guide slots for making a rear femoral resecting operation and tapering subsidiary resections.

## BRIEF DESCRIPTION OF THE DRAWINGS

Further characteristics and advantages of the apparatus for resecting the femoral and tibial bones for fitting knee articulation prostheses to the resected bones will become more apparent hereinafter from the following detailed disclosure of a preferred, though not exclusive, embodiment thereof, which is illustrated, by way of an indicative but not limitative example, in the figures of the accompanying drawings, where:

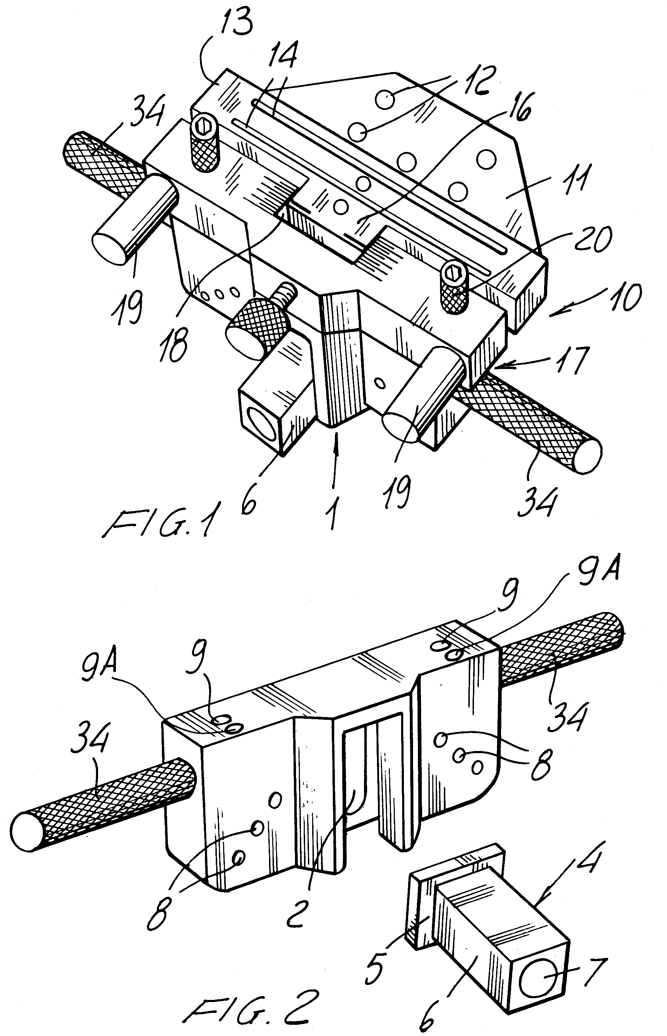

Figure 1 shows a central body included in the resecting apparatus according to the present invention, which central body is coupled to a sliding guide;

Figure 2 is another view of the central body, bearing a slide element;

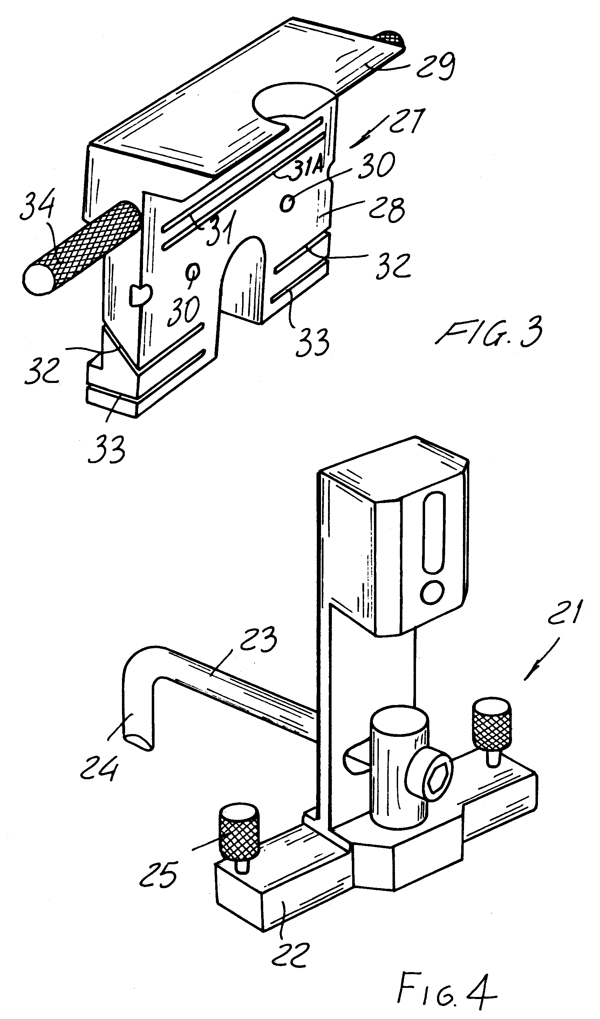

Figure 3 is a perpsective view illustrating a dihedral body included in the femoral bone resecting apparatus according to the present invention;

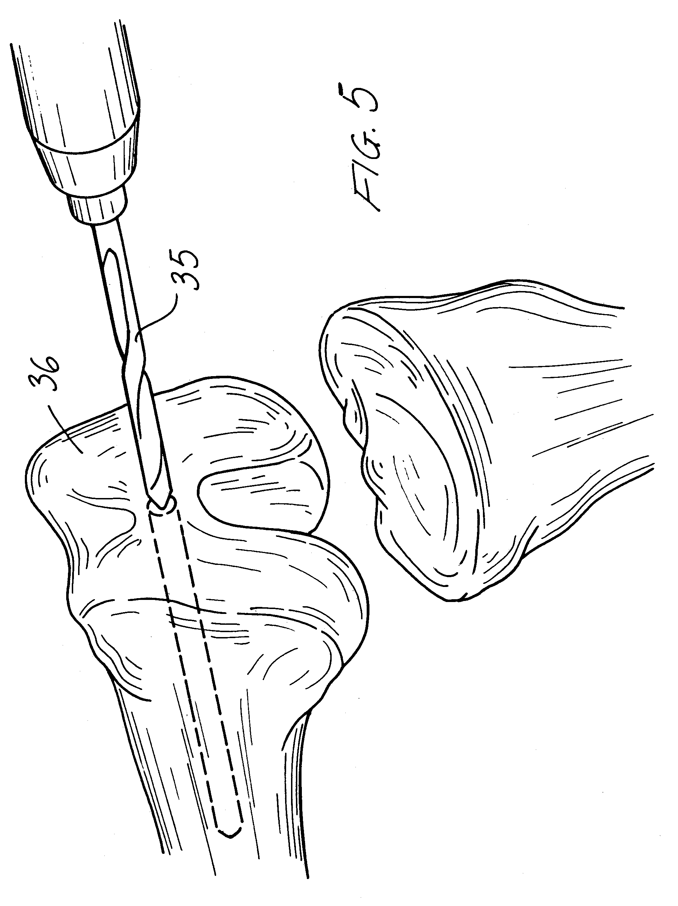

Figure 4 shows a top body which can be coupled to the mentioned central body;

Figure 5 shows a possible procedure for drilling the femoral epiphysis at the diaphysis axis of the femoral bone;

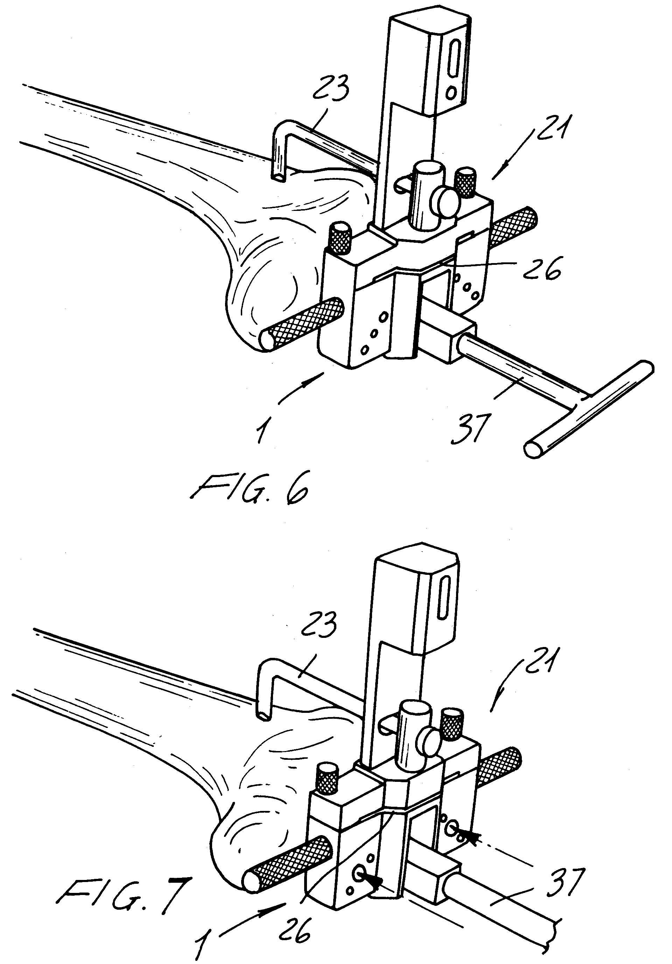

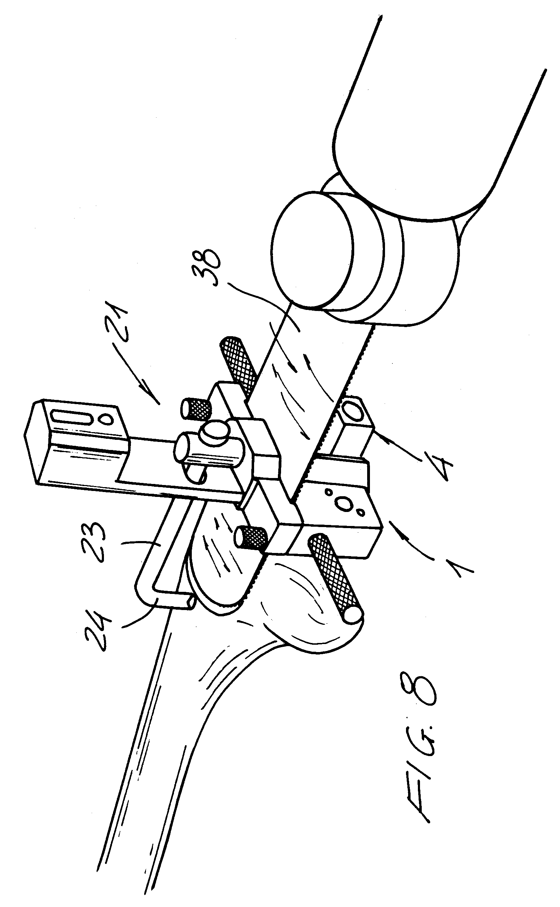

Figures 6, 7 and 8 show a possible operating procedure followed for resecting at the front thereof the femoral bone;

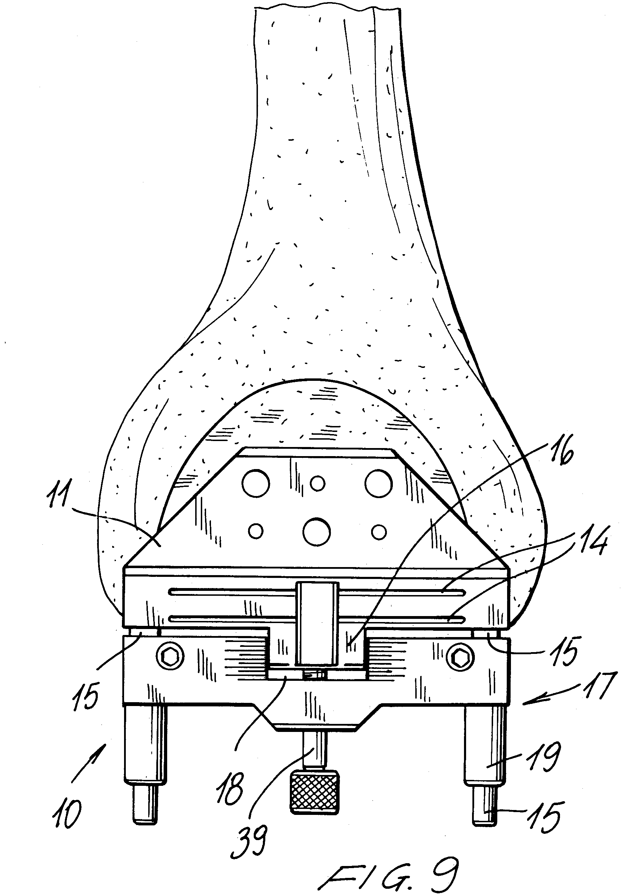

Figures 9, 10 and 11 show a possible procedure for distally resecting the femoral bone;

Figure 12 shows a possible procedure for applying the dihedral shaped body on the distal portion of the femoral bone;

Figure 13 schematically illustrates a rear resection operation of the femoral condyles and a subsidiary operation for making tapering resections;

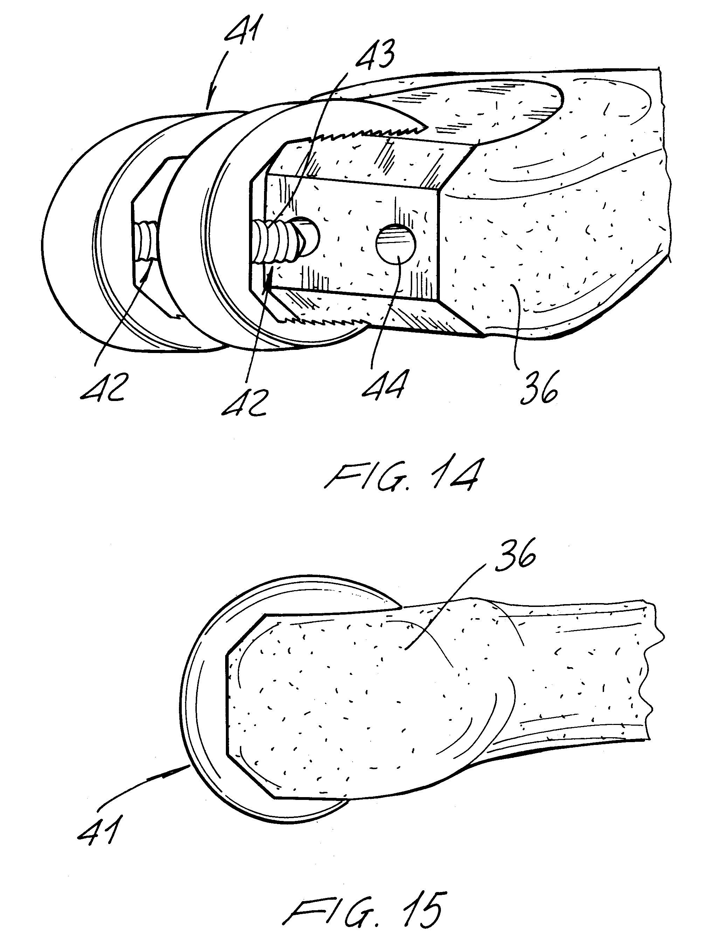

Figure 14 shows a possible procedure for applying a femoral prosthesis on the femoral bone;

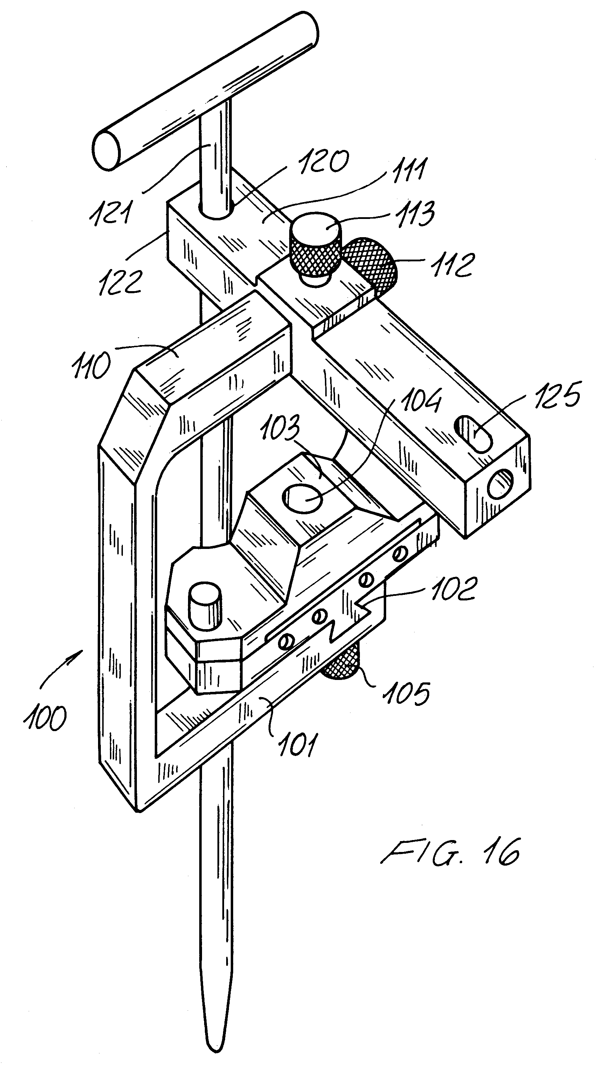

Figure 15 is a side view showing the prosthesis applied on the femoral bone;

Figure 16 is a perspective view illustrating an endomedullar tibial resection guide;

Figure 17 illustrates the above mentioned guides by a front view thereof; and

Figure 18 is a side elevation view illustrating that same guide.

## DESCRIPTION OF THE PREFERRED EMBODIMENTS

With reference to the figures of the accompanying drawings, the apparatus for resecting the femoral and tibial bones for fitting knee articulation prostheses to the resected bones, according to the present invention, comprises a central body 1, having a substantially parallelepipedal shape, transversely of which there is formed a throughgoing slot 2.

On this slot 2 there is provided a projecting frame 3, which is opened at the bottom thereof and is adapted to define sliding guides for a slide element 4 including a rectangular plate 5 therefrom a square or rectangular rod 6 extends, said rod having any suitable length.

Through said rod and plate there is formed a hole 7, which extends along an axis which is slightly slanted with respect to the longitudinal axis of said rod.

Owing to this provision, it will be possible, by reversing the position of the plate in its related guides, to operate either on the right articulation or on the left articulation of the leg.

In this connection it should be mentioned that, on the two sides of the mentioned projecting frame, there are diagonally provided throughgoing holes 8 and that, on the top wall of the central body there are formed, at the end portions thereof, corresponding threaded counterbores 9, as well as corresponding not threaded counterbores 9a.

With the mentioned central body, a sliding guide 10 cooperates which is provided with a front trapezoidal portion 11 including cross throughgoing holes 12 and comprising a plate which is arranged cantilever-wise with respect to a square rod 13.

The latter is provided, in turn, with two longitudinal slots 14, which are perpendicular to the above mentioned plate, and, oppositely extending with respect to said plate, two side sliding pins 15, as well as a central ridge 16, having a rectangular cross-section, on the axis of which there is formed a further counterbore, adapted to suitably restrain the end portion of the stem of an operating screw element.

With the mentioned front portion, a rear portion 17 cooperates, said rear portion 17 being also constituted by a square rod which, at the rear portion thereof, is provided with a central ridge, including a throughgoing threaded hole, to which a frontal gap 18 corresponds, adapted to operate as a seat for slidably housing the ridge of said front portion.

The rod forming said rear portion is moreover provided, at the end portions thereof, with corresponding throughgoing holes, extended by bush elements 19, as well as with cross holes in which locking screws 20 can be engaged, centering pins being moreover provided for engaging in the mentioned holes 9a, which pins have not been shown.

The mentioned top body, generally indicated at the reference number 21, comprises a base or bottom plate 22, and is provided with an abutment lug 23, having a right-angle bent end portion 24.

From the bottom plate, moreover, further centering pins extend (not shown) which can be engaged in corresponding recesses or holes 9a provided on the top wall of the central body 1, as well as throughgoing holes adapted to guide corresponding screws 25 engaged in the threaded holes 29.

In this connection, it should be moreover added that said bottom plate is furthermore provided, at the end portions thereof, with bottom ridges so as to define, with the bearing wall of the central body, a middle slot 26.

The dihedral body, generally indicated at the reference number 27, comprises a substantially rectangular plate 28, on the top whereof there is provided a cantilever perpendicular wall 29 having, preferably, a beveled edge.

Through the thickness of this plate, at the central portion of which there is provided a perimetrical gap, there are formed two throughgoing holes 30, a middle top slot 31, upward slanted, a middle slot 31a downward slanted, two downward slanted side slots 32, and two side bottom slots 33, which are perpendicular to said plate.

As shown, the above mentioned central body and dihedral body are provided with side handles 34, for properly arranging said body at a desired location.

For performing the femoral resection operations, after having made, by a suitable drill 35, a hole through the femoral epiphysis 36, at the diaphysis axis, the central body 1, thereon is assembled the top body 21, is caused to rest on the femoral epiphysis, by using the slide element 4 and a rod 37, as well as the right-angle end portion of the abutment element 23.

Then, by means of nails or the like elements, as engaged in the holes 8, the central body is restrained to the femoral epiphysis and, by a suitable vibrating element 28, the front portion of the femnoral bone is resected.

Then, the top body 21 is disengaged from the central body 1, and on said central body there is affixed said sliding guide 10 by means of the above mentioned locking screws 20.

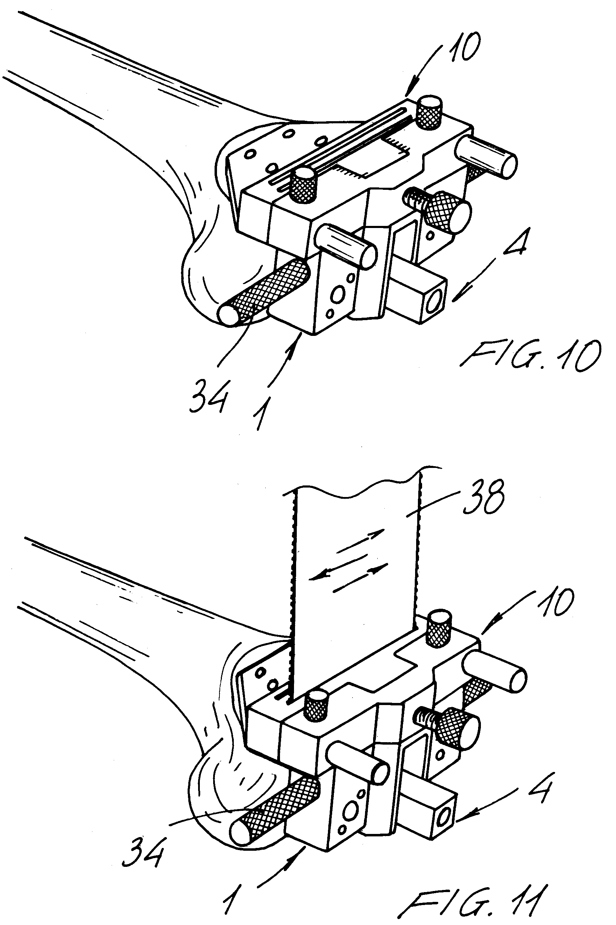

This sliding guide will allow, by using one of the longitudinal slots 14, to perform the distal cross resection of the femoral bone, by means of a suitable resecting blade (as shown in figure 11).

In this connection, it should be pointed out that, by operating on a provided central screw 39, it will be possible to micrometrically displace the front portion 11 with respect to the rear portion 17 of the sliding guide, so as to perform the resecting operation at the most appropriate position.

Then, the central body and sliding guide are removed, and on the distal portion of the femoral. bone the shaped dihedral body 27 is restrained by using nails or the like.

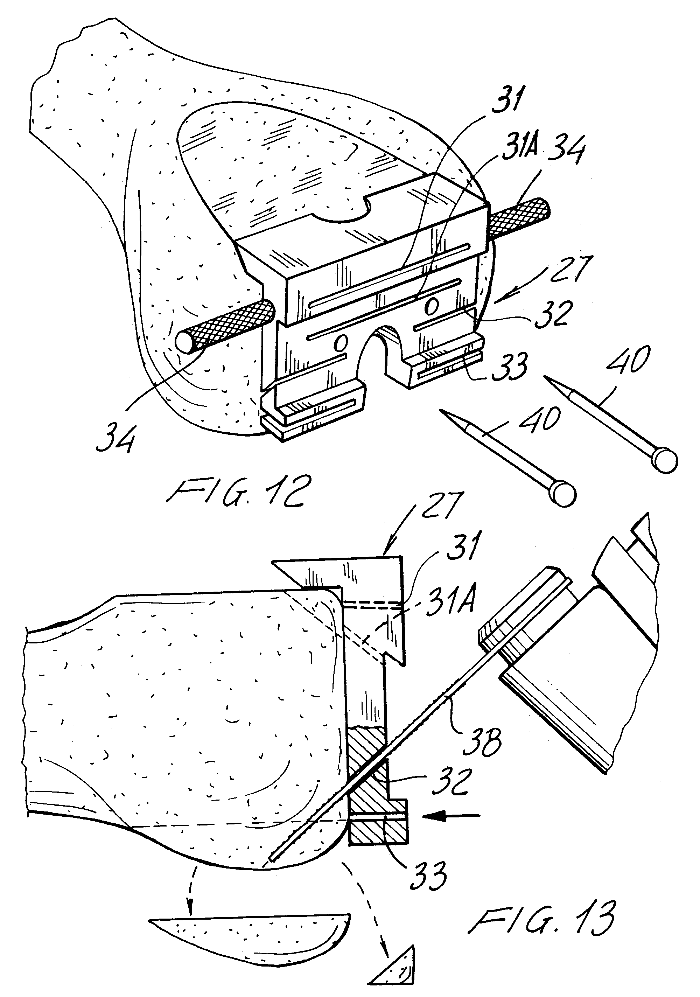

Through the slot 33, formed through said dihedral body, the rear portion of the femoral bone will be then resected, whereas, by means of the slots 31, 31a and 32 it will be possible to make subsidiary tapering resections, as is shown in figure 13.

After having suitably processed, by the above mentioned resecting operations, the epiphysis 36 of the femoral bone, the articulation prosthesis, generally indicated at the reference number 41 can be properly applied, by locking it to the bone by force fitting pins 42, provided with annular ridges 43, in corresponding holes 44.

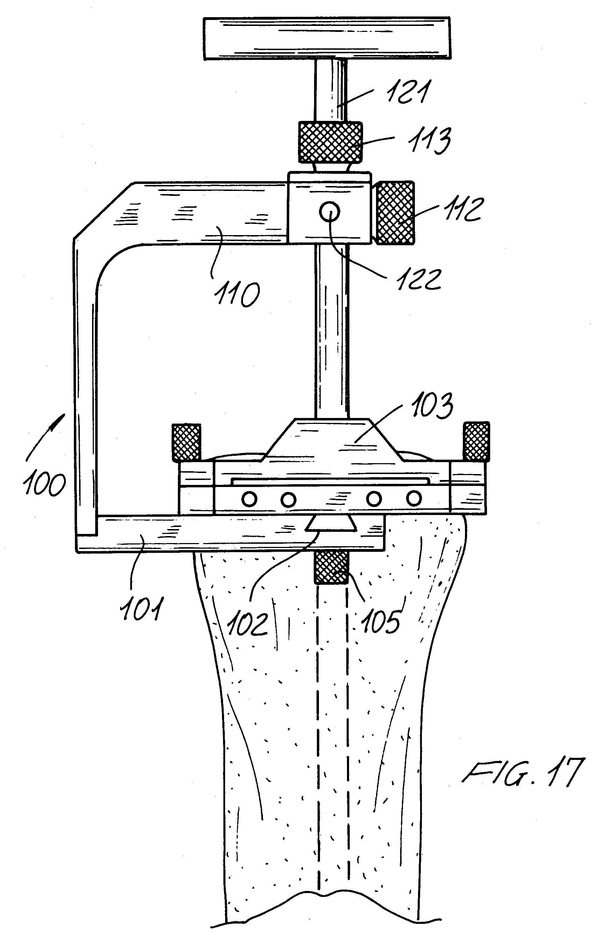

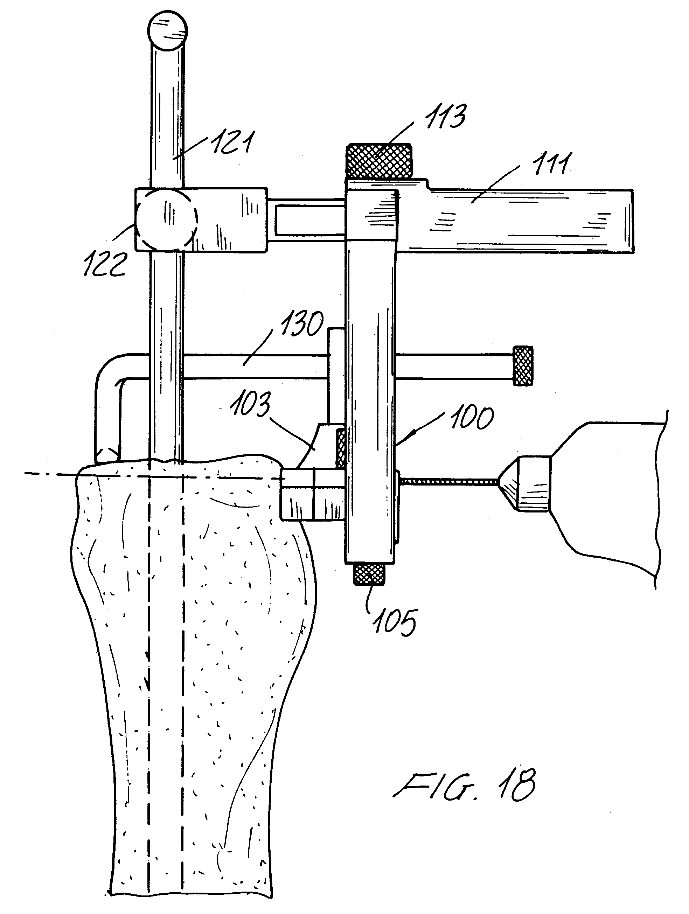

With reference to figures 16 to 18 an apparatus will be hereinafter discloses for resecting the tibial bone, which will be simply called endomedullar tibial resecting guide.

The above mentioned guide substantially comprises a central body, generally indicated at the reference number 100, which has a C-shape and defines a bottom central leg 101, provided with a dovetail guide 102, thereon a resecting guide 103 can be applied, said resecting guide 103 being provided with a hole 104 for mounting a sensing element.

More specifically, the resecting guide 103 can be properly located by means of a bottom screw 105 for coupling said guide to the bottom leg 101.

The guide body 100 defines moreover a top leg 110 which is connected to a side rod 111 by means of a horizontal pin for performing a front-rear movement, and which can be driven by an adjusting screw 112.

A top screw 113 is moreover provided for preventing any turning movement about to the above mentioned cross pin coupled to the screw 112.

The side rod 111 is provided with a guide hole 120 in which an endomedullar nail 121 can be engaged, a set front screw 122 being provided for locking said nail in its position.

The side rod is further provided with a control hole 125, therein a control rod can be engaged.

For performing a tibial bone resecting operation, the endomedullar nail 121, thereon there is mounted said guide body, will be force-introduced into the tibial medullar channel, by impacting said nail from the tibial bone top side.

After having properly located the endomedullar tibial nail 121, on the dovetail element 102 formed on the top central leg 101 a resecting guide 103 will be located, said resecting guide 103 supporting a related sensing element 130, and the assembly will be locked by the bottom set screw 105.

For performing a tibial bone resection as precise as possible, the apparatus has the feature that the vertical position along the endomedullar nail 121 can be easily adjusted, by operating the set screw 122, which locks the nail 121 in the hole 120, in order to provide a proper resecting height.

Likewise, the screw 122 prevents the endomedullar nail from rotating about a vertical axis, in order to easily resect even regions which can not be easily accessed by the swinging saw.

The front-rear displacement of the guide is assured by the horizontal pin, and is obtained, more specifically, by operating the screw 112 which allows the operator to easily fit the different front-rear size of the tibial bone.

The turning movement about the cross horizontal pin, as controlled by the screw 112, is locked by means of the top screw 113, or in a neutral position, that is with the resection or cut performed in a perpendicular direction to the mechanical axis of the leg, or with a variable slope with respect to the front plane, by operating on the screw 113 which is adapted to lock the slide element at the desired position.

The adjustable assembling of the guide is obtained by using an adjusting or control rod which is engaged in the control holes 125 so as to allow the aligning angle of the guide with respect to the leg mechanical axis to be easily controlled.

The rod, which can slide in the hole, will also allow the aligning with the central portion of the femoral bone head to be easily controlled.

From the above disclosure and the figures of the accompanying drawings it should be apparent that the invention fully achieves the intended aim and objects.

While the invention has been disclosed and illustrated with reference to a preferred embodiment thereof, it should be apparent that the disclosed embodiment is susceptible to several modifications and variations, all of which will come within the spirit and scope of the appended claims.

## Claims

1- An apparatus for resecting the femoral and tibial bones for fitting knee articulation prostheses to the resected bone,characterized in that said apparatus comprises a slide bearing central body,therewith a sliding guide,for distally resecting the femoral condyles, and a top body,for performing the front femoral resections cooperate,there being moreover provided a dihedral body adapted to form guide slots for making a rear femoral resecting operation and subsidiary tapering resections.

2- An apparatus according to claim 1,characterized in that said central body has a substantially parallelepipedal shape and is provided with a transversal throughgoing slot encompassed by a projecting frame,which is open at the bottom thereof and is adapted to define sliding guides for a slide element comprising a rectangular plate forming a base element for a square or rectangular cross-section rod,a hole being formed along said plate and rod extending along an axis slightly slanted with respect to the longitudinal axis of said rod.

3- An apparatus,according to claims 1 and 2,characterized in that on the two sides of said projecting frame there are formed diagonal throughgoing holes and that, on the top wall of said central body,there are formed corresponding end threaded counterbores.

4- An apparatus according to one or more of the preceding claims,characterized in that said sliding guide is provided with a trapezoidal front portion provided with cross throughgoing holes and comprising a plate cantilever wise arranged with respect to a square cross-section rod provided with two longitudinal slots perpendicular to said plate and oppositedly extending with respect to said plate,two side sliding pins as well as a rectangular cross-section central ridge on the axis of which there is provided a counterbore adapted to restrain one end of the stem of an operating screw element.

5- An apparatus according to one or more of the preceding claims,characterized in that with the front portion of said sliding guide a rear portion cooperates-,said rear portion also substantially comprising a square cross-section rod provided,at the rear thereof,with a central ridge including a threaded throughgoing hole and mating with a frontal gap adapted to slidably house said ridge of said front portion-,said rod,forming said rear portion being moreover provided with corresponding end throughgoing holes extended by bush elements as well as with cross holes therein locking screws are engaged.

6- An apparatus,according to one or more of the preceding claims,characterized in that said top body is provided with a base plate and an abutment lug,including a right angle bent end portion,from said base plate centering pins extending which can be engaged in corresponding seats formed on the top wall of said central body, as well as throughgoing holes for engaging therein corresponding screws engaging in threaded holes formed in said central body,said base plate being moreover provided,at the ends thereof,with bottom ridges adapted to define,with the bearing wall of said central body,a middle slot.

7- An apparatus,according to one or more of the preceding claims,characterized in that said dihedral body comprises a substantially rectangular plate on the top of which there is provided a cantilever perpendicular wall having a beveled edge,through the thickness of said plate,at the central portion whereof there is formed a perimetrical gap,there being provided two throughgoing holes,a middle top slot,upward sloped,two side slots,downward sloped,and two bottom side slots which are perpendicular to said plate.

8- An apparatus,according to one or more of the preceding claims,characterized in that said central body and dihedral body are provided with side handles.

9- An apparatus according to one or more of the preceding claims,characterized in that said apparatus further comprises an endomedullar tibial resection guide having a substantially C shaped guide body and provided,on the bottom leg thereof,with means for coupling a resecting guide and,on the top leg thereof,with a side rod for coupling and guiding an endomedullar nail.

10- An apparatus according to one or more of the preceding claims,characterized in that said bottom leg defines a dovetail guide element therein a resecting guide can be engaged,a bottom screw being moreover provided for locking said resecting guide in said dovetail guide element.

11- An apparatus according to one or more of the preceding claims,characterized in that said side rod is coupled to said top leg by a horizontal and cross pin associated with a driving screw adapted to lock said side rod and drive said side rod in a front-rear direction with respect to the head portion of the tibial bone.

12- An apparatus according to one or more of the preceding claims,characterized in that said apparatus further comprises a top screw associated with said side rod and engaging with said pin for preventing said side rod from turning about said pin.

13- An apparatus according to one or more of the preceding claims,characterized in that said side rod is provided with a throughgoing hole for engaging said endomedullar nail,in said hole a further screw engaging for locking said side rod with respect to said endomedullar nail and preventing said side rod from rotating with respect to said endomedullar nail.

FIG.1

FIG.2

FIG. 3

FIG. 4

8

FIG. 5

FIG. 6

FIG. 7

FIG. 8

FIG. 9

FIG. 10

FIG. 11

31

31A 34

27

32

33

40

40

34

FIG. 12

27

31

31A

38

32

33

FIG. 13

FIG. 14

FIG. 15

FIG. 16

*121*

*113*

*112*

*110*

*122*

*103*

*100*

*101*

*102*

*105*

FIG. 17

FIG. 18